# EUROPEAN PATENT APPLICATION

(11) **EP 4 458 434 A1**
(43) Date of publication of application: **06.11.2024**
(21) Application number: 22916554.3
(22) Date of filing: 14.12.2022
(51) Int. Cl.: A63B 24/00, H04W 4/60, A63B 71/06

(54) **APPARATUS AND METHOD FOR PROVIDING EXERCISE SEQUENCE**

(30) Priority: 28.12.2021 KR 20210190374; 28.12.2021 KR 20210190375
(71) Applicant: DRAX Inc., Anyang-si, Gyeonggi-do 14086 (KR)
(72) Inventor: YOO, Seon Kyung, Seoul 08251 (KR)
(74) Representative: Bianchetti & Minoja with Trevisan & Cuonzo IPS SRL
(86) International application number: PCT/KR2022/020367
(87) International publication number: WO 2023/128410

(57) **Abstract**

As a preferred embodiment of the present disclosure, a method of providing an exercise sequence includes grouping, by a grouping unit, exercise equipment in a smart gym into a plurality of exercise groups, and, by an exercise sequence generating unit, selecting at least one piece of exercise equipment belonging to each of the plurality of exercise groups and generating an exercise sequence including a use order of the at least one piece of exercise equipment and an exercise program for each of the at least one piece of exercise equipment.

## Description

### Technical Field

The present disclosure relates to a method of providing an exercise sequence to a user according to the user's physical condition.

### Background Art

Weightlifting equipment is provided in various forms depending on the body part of which muscle strength is to be increased or the purpose of use, and is mainly used to train the upper body and the lower body by using hands or feet. A user may perform an exercise by moving a selected weight through the structure of the weightlifting equipment.

However, it may be difficult for the user to determine the necessary exercise equipment and the level of exercise load according to the user's physical development status. It may also be difficult for the user to create an exercise plan with high exercise effectiveness according to the user's physical characteristics.

### Disclosure

### Technical Problem

A preferred embodiment of the present disclosure provides an exercise sequence that includes information about exercise equipment, exercise load, and user order of the exercise equipment, which is necessary for a user.

A preferred embodiment of the present disclosure provides an exercise sequence to evenly develop a user's entire body muscles according to the user's cumulative exercise records, resting state, and physical condition.

### Technical Solution

As a preferred embodiment of the present disclosure, an apparatus for providing an exercise sequence includes a grouping unit for grouping exercise equipment in a smart gym into a plurality of exercise groups, and an exercise sequence generating unit for generating an exercise program by selecting at least one piece of exercise equipment belonging to each of the plurality of exercise groups and generating an exercise sequence including a use order of the exercise equipment belonging to the exercise program.

### Advantageous Effects

As a preferred embodiment of the present disclosure, an apparatus for providing an exercise sequence has the effect of evenly developing the muscles of the entire body by providing a user with an exercise sequence that includes exercise equipment and exercise programs tailored to each user's individual characteristics.

### Description of Drawings

FIG. 1 is a diagram of a smart gym system in which an apparatus for providing an exercise sequence is used, as a preferred embodiment of the present disclosure.
FIG. 2 is a diagram of an example of exercise equipment in which a method of providing an exercise sequence in a smart gym is implemented, as a preferred embodiment of the present disclosure.
FIG. 3 is an internal configuration diagram of exercise equipment and a smart gym server in a smart gym system in which a method of providing an exercise sequence is implemented, as a preferred embodiment of the present disclosure.
FIG. 4 is an internal configuration diagram of an apparatus for providing an exercise sequence, as a preferred embodiment of the present disclosure.
FIG. 5 is a diagram illustrating an example of providing an exercise sequence, as a preferred embodiment of the present disclosure.

### Best Mode

As a preferred embodiment of the present disclosure, an apparatus for providing an exercise sequence includes a grouping unit for grouping exercise equipment in a smart gym into a plurality of exercise groups, and an exercise sequence generating unit for generating an exercise program by selecting at least one piece of the exercise equipment belonging to each of the plurality of exercise groups and generating an exercise sequence including a use order of the exercise equipment belonging to the exercise program.

As a preferred embodiment of the present disclosure, the grouping unit performs grouping based on exercise target muscles, and in this case, further refers to the joint movement or the number of joints used during exercise for each exercise target muscle.

As a preferred embodiment of the present disclosure, the grouping unit performs grouping based on body parts.

As a preferred embodiment of the present disclosure, the exercise sequence generating unit selects the at least one piece of exercise equipment based on a user's physical condition, and the user's physical condition is analyzed by personal maximum weight (PMW) or maximum oxygen intake (VO₂Max).

As a preferred embodiment of the present disclosure, the exercise sequence generating unit generates the exercise program by setting an exercise intensity for each of the at least one piece of exercise equipment belonging to the plurality of exercise groups based on the physical condition of the user, and the exercise intensity is set based on the exercise load and the number of repetitions.

In a preferred embodiment of the present disclosure, the exercise sequence generating unit generates at least one exercise sequence in a preset time unit so that the user's entire body muscles are developed evenly.

As a preferred embodiment of the present disclosure, a method of providing an exercise sequence includes grouping exercise equipment in a smart gym into a plurality of exercise groups by a grouping unit, and selecting at least one piece of exercise equipment belonging to each of the plurality of exercise groups and generating an exercise sequence including a use order of the at least one piece of exercise equipment and an exercise program for each of the at least one piece of exercise equipment by an exercise sequence generating unit.

### Mode for Invention

Hereinafter, some embodiments of the present disclosure are described in detail with reference to the attached drawings so that those skilled in the art can easily practice the present disclosure.

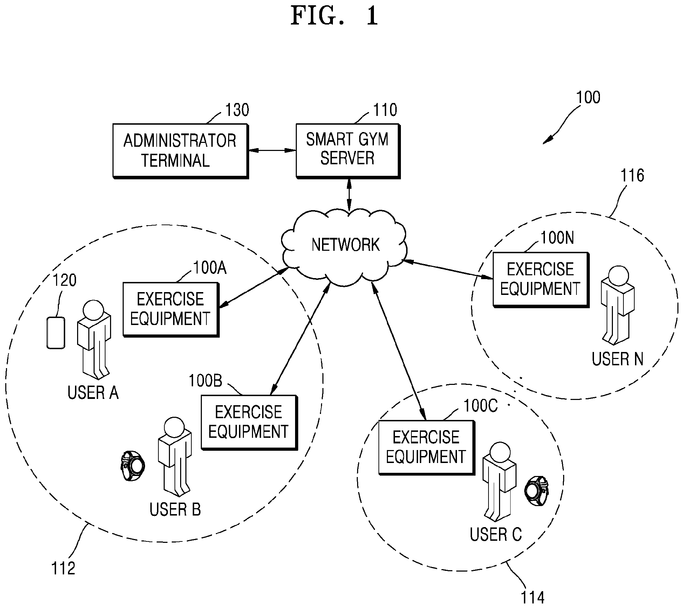

FIG. 1 is a diagram of a smart gym system in which an apparatus for providing an exercise sequence is used, as a preferred embodiment of the present disclosure.

A smart gym system 100 includes a smart gym server 110, at least one piece of exercise equipment 100A, 100B, 100C, and 100N, at least one user terminal 120, and an administrator terminal 130.

In FIG. 1, the smart gym server 110 may communicate with a first smart gym 112, a second smart gym 114, and an nth smart gym 116 at physically different locations, and may exchange data with at least one piece of exercise equipment 100A and 100B in the first smart gym 112, at least one piece of exercise equipment 100C in the second smart gym 114, and at least one piece of exercise equipment 100N in the nth smart gym 116.

As a preferred embodiment of the present disclosure, a smart gym is a physical space where a user's record of using exercise equipment is provided to the smart gym server 110 and an exercise prescription tailored to the user is provided by the smart gym server 110 which learns and analyzes the user's record. The smart gym may be implemented as a fitness center, a gym, or a space equipped with exercise equipment.

Users USER A, USER B, USER C, and USER N who come to the smart gym to exercise may enter the smart gym after verifying their identity when entering the smart gym. For example, a user may enter the smart gym after the user's membership is confirmed by tagging the user terminal 120 to an unmanned terminal, such as a kiosk, at the smart gym entrance, using near field communication (NFC) or radio frequency identification (RFID), or may enter the smart gym after the user's membership is confirmed by verifying biometric information, such as facial recognition, on the unmanned terminal.

Information about the user whose membership has been confirmed may be transmitted from the smart gym server 110 to at least one piece of the exercise equipment 100A, 100B, 100C, and 100N through a network. For example, the smart gym server 110 may transmit the information about the user to the exercise equipment to which the user has tagged the terminal 120. In a preferred embodiment of the present disclosure, the information about the user is also referred to as user data and includes at least some or all of the user's gender, age, weight, height, BMI, and body fat percentage.

The smart gym server 110 may provide a first user USER A and a second user USER B who use at least one piece of the exercise equipment 100A and 100B in the smart gym 112, respectively, with an exercise method, an exercise intensity, and an exercise sequence, which are tailor to each user. Additionally, the smart gym server 110 may provide values, such as a target weight and a recommended usage speed, for each piece of the exercise equipment 100A and 100B. Additionally, the smart gym server 110 may receive exercise records of the first user USER A and the second user USER B using the exercise equipment 100A and 100B, respectively. The smart gym server 110 may further receive health information or log information, such as a user's heart rate, blood pressure, pulse, etc., from the user terminal 120.

The smart gym server 110 may be implemented in the form of a cloud server. The smart gym server 110 may integrate and manage information collected from each exercise equipment in smart gyms located at different locations. For example, the smart gym server 110 may integrate and manage the details of the first user's use of the exercise equipment in the smart gym 112 at a first location and the details of the first user's use of the exercise equipment in the smart gym 114 at a second location.

As a preferred embodiment of the present disclosure, at least one piece of the exercise equipment 100A, 100B, 100C, and 100N may be stretching equipment, weightlifting equipment, or cardio equipment. The weightlifting equipment includes a free weight and a weight machine. At least one piece of the exercise equipment 100A, 100B, 100C, and 100N provides a suitable exercise guide to the user through a display attached to the exercise equipment or a display capable of wired or wireless communication with the exercise equipment. For example, the stretching equipment provides an exercise guide related to stretching for the user to use through a smart mirror capable of wired or wireless communication with the exercise equipment. However, it is not limited thereto, and the exercise guide may be provided using various output methods, such as speakers and vibration.

At least one piece of the exercise equipment 100A, 100B, 100C, and 100N may perform wired or wireless communication with the smart gym server 110, the user terminal 120, and the administrator terminal 130.

As a preferred embodiment of the present disclosure, the user terminal 120 may be implemented in the form of a smartphone, a smartwatch, a handheld device, a wearable device, etc. Additionally, applications for using the smart gym system 100 may be installed on the user terminal 120. The user terminal 120 may receive exercise sequence information, etc. from the smart gym server 110. The exercise sequence refers to an exercise plan created considering the user's physical condition and exercise ability. The exercise sequence includes information, such as a list of exercise equipment to be used by a user, a target weight, and the number of uses of each exercise equipment.

When using at least one piece of the exercise equipment 100A, 100B, 100C, and 100N in the smart gym system 100, the user may use the terminal 120 to communicate with the exercise equipment through tagging, such as NFC or RFID, or may use the user's physical characteristics to verify the user's identity. Once the user's identity is verified, the smart gym server 110 may transmit user data to the exercise equipment tagged by the user.

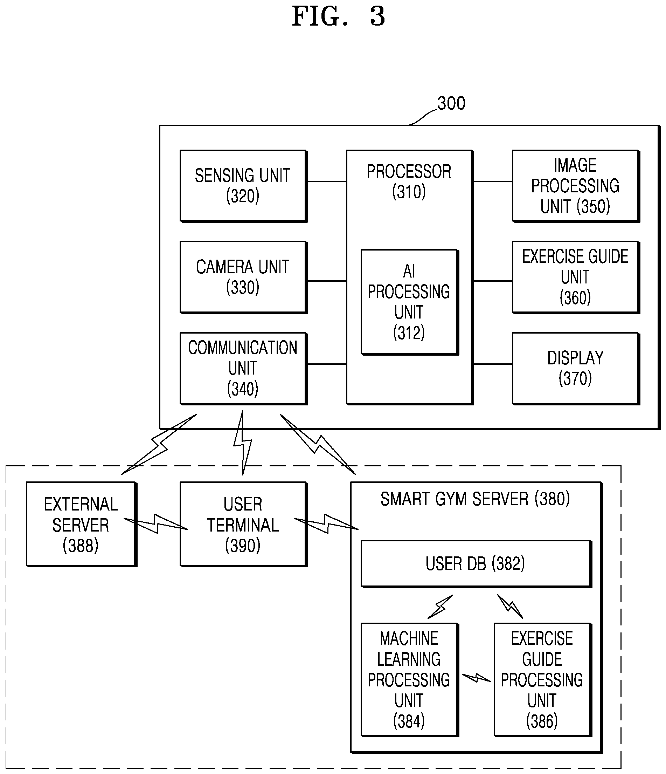

FIG. 3 is an internal configuration diagram of exercise equipment and a smart gym server in a smart gym system in which a method of providing an exercise sequence is implemented, as a preferred embodiment of the present disclosure.

As a preferred embodiment of the present disclosure, exercise equipment 300 in a smart gym may communicate with a smart gym server 380, a user terminal 390, and an external server 388.

As a preferred embodiment of the present disclosure, the exercise equipment 300 includes a processor 310, a sensing unit 320, a communication unit 340, an exercise guide unit 360, and a display 370. In addition, the exercise equipment 300 may further include a camera unit 330 and an image processing unit 350. The processor 310 may further include an Al processing unit 312, if necessary.

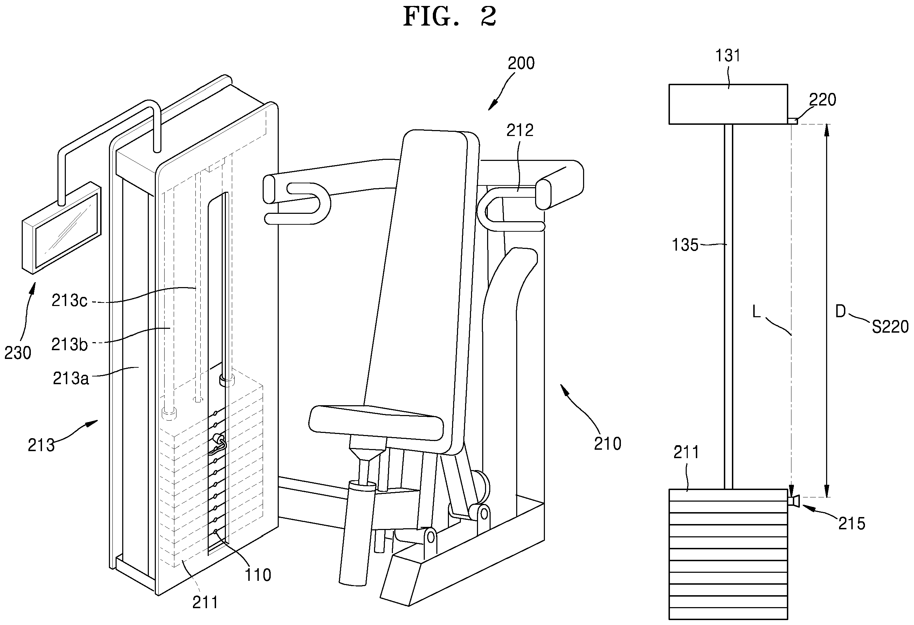

Referring further to FIG. 2, a shoulder press 200 represents an example of the exercise equipment 300 used in a smart gym. The exercise equipment 300 is described with reference to FIGS. 2 and 3.

The sensing unit 220 may be installed on a frame structure 213 of a body 210 of the shoulder press 200. The frame structure 213 includes a base frame 213a, a guide rail 213b, and a connection line 213c. The sensing unit 220 radiates a laser beam toward a pin structure 215, receives the reflected laser beam, and measures the distance D (S220) from the sensing unit 220 to the pin structure 215 in real time or in a preset time unit. The sensing unit 220 may detect at least one of the position, the moving speed, and the moving direction of a weight member 211 selected by the pin structure 215 in real time. In addition, when the user pushes a handle 212 of the shoulder press 200 to move a weight plate, the sensing unit 220 may measure the distance D (S220) to the pin structure 215 inserted into the weight plate and may detect a movement trace based thereon.

The communication unit 340 may receive user input through the display unit 230 or exchange user data with a user DB 382 of the smart gym server 380. The communication unit 340 may also communicate with the external server 388.

The exercise guide unit 360 may provide the user with information, such as user data received from the smart gym server 380, a target weight of the exercise equipment, a movement speed guide of the exercise equipment, a breathing guide when using the exercise equipment, and an exercise sequence. The exercise guide unit 360 displays the exercise sequence received from the smart gym server 380 on the display 370.

The smart gym server 380 includes the user DB 382, a machine learning processing unit 384, and an exercise guide processing unit 386. The user DB 382 stores and manages user data. The user data includes the user's gender, age, weight, height, BMI, and body fat percentage. The smart gym server 380 may calculate an estimated value of a specific muscle strength based on the user data, as shown in Equation 1. An example of the specific muscle strength may be a grip strength.

In Equation 1, A, B, C, D, and E represent preset values.

The smart gym server 380 pre-stores percentile information on relative grip strength calculated through Equation 1. Table 1 shows the percentiles of relative grip strength for adult men, and Table 2 shows the percentiles for relative grip strength for adult women.

**[Table 1]**

| | Age | | | | |
|---|---|---|---|---|---|
| Percentile | 19-29 | 30-39 | 40-49 | 50-59 | 60-69 |
| 90 | **.* | ... | | | **.* |
| ... | | | | | |
| 50 | 57.2 | | 56.1 | **.* | **.* |
| 40 | **.* | 55.9 | **.* | 53.8 | 49.2 |
| ... | | | | | |
| 10 | **.* | ... | | | **.* |

**[Table 2]**

| | Age | | | | |
|---|---|---|---|---|---|
| Percentile | 19-29 | 30-39 | 40-49 | 50-59 | 60-69 |
| 90 | **.* | ... | | | 47.5 |
| ... | | | | | |
| 50 | **.* | 46.3 | 43.8 | **.* | **.* |
| 40 | 40.3 | **.* | **.* | **.* | 34.1 |
| ... | | | | | |
| 10 | 31.7 | ... | | | **.* |

In addition, as shown in Table 3, the smart gym server 380 has a mapping table that stores PMWₑₛₜᵢₘₐₜₑ percentile information generated based on personal maximum weight (PMW) data obtained from the population for each exercise equipment. Table 3 shows PMWₑₛₜᵢₘₐₜₑ percentile information for leg extension exercise equipment.

**[Table 3]**

| PMWₑₛₜᵢₘₐₜₑ percentile | Population PMWₑₛₜᵢₘₐₜₑ (kg) |
|---|---|
| PMW90 | 103.3 |
| ... | ... |
| PMW50 | 67.3 |
| PMW40 | 66.5 |
| ... | ... |
| PMW10 | 24.6 |

In a preferred embodiment of the present disclosure, the PMW refers to a muscle strength that an individual can exert against the resistance of a weight with maximum effort. Examples of the PMW include 1RM, 4RM, etc. The PMWₑₛₜᵢₘₐₜₑ refers to an estimated value of the user's PMW based on the estimated muscle strength value calculated through Equation 1.

The smart gym server 380 may determine the PMWₑₛₜᵢₘₐₜₑ for each exercise equipment. The smart gym server 380 determines the PMWₑₛₜᵢₘₐₜₑ of the exercise equipment to be used by the user based on the estimated muscle strength value and PMWₑₛₜᵢₘₐₜₑ percentile information. Additionally, based on PMWₑₛₜᵢₘₐₜₑ, an initial target weight of the exercise equipment to be used by the user is automatically set.

For example, when an estimated muscle strength value of a 32-year-old man is 55.9 and a percentile value to which the estimated muscle strength value belongs is 40, the corresponding PMWₑₛₜᵢₘₐₜₑ percentile value is 40, that is, the leg extension PMW of the 32-year-old man is estimated to be 66.5 kg. As a preferred embodiment of the present disclosure, the smart gym server 380 pre-stores PMWₑₛₜᵢₘₐₜₑ percentile information for each exercise equipment as shown in Table 1.

The exercise guide processing unit 386 sets an initial target weight of each exercise equipment based on PMWₑₛₜᵢₘₐₜₑ. In this case, the exercise guide processing unit 386 may set the initial target weight thereof according to the exercise intensity or exercise purpose. The exercise intensity may be determined according to the exercise volume to be provided in the exercise sequence in a preset time unit. For example, the preset time unit is one week. When exercise load to be applied to each muscle for a week is determined, the exercise guide processing unit 386 may set the exercise intensity according to the rest period for each muscle and the cumulative exercise volume in the preset time unit. The exercise volume is the product of weight, number of repetitions, and sets.

When the exercise intensity is low, the weight may be set to a% of the PMWₑₛₜᵢₘₐₜₑ, when the exercise intensity is intermediate, the weight may be set to b% of the PMWₑₛₜᵢₘₐₜₑ, and when the exercise intensity is high, the weight may be set to c% of the PMWₑₛₜᵢₘₐₜₑ. As an example, a may be set to 20 to 40, b may be set to 40 to 60, and c may be set to 60 to 80. However, this is only an example and various modifications are possible.

The exercise guide processing unit 386 may provide an initial target weight of the exercise equipment to be used by the user to the exercise guide unit 360 of the exercise equipment 300, and the exercise guide unit 360 may display the initial target weight of the exercise equipment to be used by the user on the display 370.

As another embodiment of the present disclosure, the smart gym server 380 may predict PMW_{individual} reflecting the user's individual objectification index. Additionally, the smart gym server 380 may update a target weight of the exercise equipment based on the predicted PMW_{individual}. More specifically, the smart gym server 380 sets the initial target weight of the exercise equipment based on PMWₑₛₜᵢₘₐₜₑ, and predicts the PMW_{individual} by reflecting the objectification index for each user when the objectification index for each user is obtained in a preset time unit. The smart gym server 380 then updates the target weight of the exercise equipment based on the PMW_{individual.}

An example of the objectification index is an exercise record, and the exercise record includes the weight of exercise equipment, the number of repetitions (reps), the number of sets, the movement trace, the movement speed, and the regularity of reps per set, each identified when using the exercise equipment.

The machine learning processing unit 384 learns and processes the objectification index including the exercise record of the exercise equipment used by the user in the smart gym. The machine learning processing unit 384 may update the PMWₑₛₜᵢₘₐₜₑ to PMW_{individual} based on the objectification index of the exercise equipment used by the user in the smart gym for a certain period. The machine learning processing unit 384 may update the PMW_{individual} to be greater than the PMWₑₛₜᵢₘₐₜₑ when the objectification index is greater than or equal to a first reference value and may update the PMW_{individual} to be less than the PMWₑₛₜᵢₘₐₜₑ when the objectification index is less than or equal to a second reference value.

Thus, even when the PMWₑₛₜᵢₘₐₜₑ for a first user and a second user who have the same gender, age, weight, height, BMI, and body fat percentage is the same, the machine learning processing unit 384 may learn and predict PMW_{mdividual} suitable for the first user and the second user for each exercise equipment used by the first user and the second user by further reflecting the individual objectification index including the exercise records of the first user and the second user.

An example of generating an objectification index by the machine learning processing unit 384 is as follows. For example, the machine learning processing unit 384 determines the completeness of the movement trace using at least some of the ascent start point, descent start point, ascending section speed V1, descending section speed V2, ascending section average speed, descending section average speed, and height H, each identified from the movement trace detected while the user was using the shoulder press 200. For example, when the ascent start point is between 0 and tₐ seconds, it is determined to be appropriate, and when the ascent start point deviates from tₐ, it is determined to be late. In addition, the completeness of the ascent start point may be given a score of high, medium, low, or 1 to 10 points according to preset criteria. Similarly, when the ascending section speed V1 is between Va and Vb according to preset criteria, it is determined to be appropriate; when ascending section speed V1 exceeds Vb, it is determined to be fast; and when the ascending section speed V1 is less than Va, it is determined to be slow. The results of determining that the ascending section speed V1 is appropriate, fast, and slow may be given scores of high, medium, low, or 1 to 10 points. As described above, the machine learning processing unit 384 may generate the objectification index from the user's exercise record.

As a preferred embodiment of the present disclosure, when using the reps as an objectification index, the machine learning processing unit 384 may determine the completeness of the reps based on the regularity between the movement traces of the total number of repetitions constituting one set and the execution time for performing the total number of repetitions constituting the one set, and may convert the degree of completeness of the reps into a number and use the same as an objectification index.

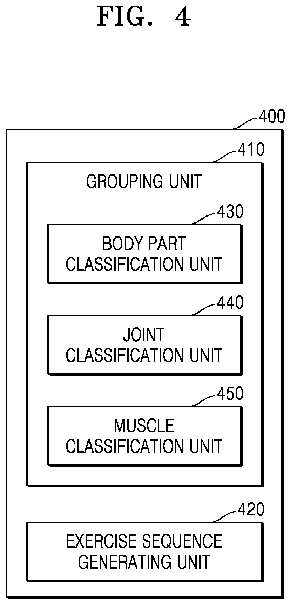

FIG. 4 is an internal configuration diagram of an apparatus 400 for providing an exercise sequence, as a preferred embodiment of the present disclosure.

The apparatus 400 for providing an exercise sequence includes a grouping unit 410 and an exercise sequence generating unit 420.

The grouping unit 410 groups exercise equipment in a smart gym into a plurality of exercise groups. The grouping unit 410 includes a body part classification unit 430, a joint classification unit 440, and a muscle classification unit 450. The smart gym server 380 stores the information shown in Table 4, and the grouping unit 410 uses the information to group the exercise equipment into a plurality of exercise groups.

**[Table 4]**

| **Exercise equipment** | **Joint Movement** | **Exercise Target Muscle** | **Muscle** | |
|---|---|---|---|---|
| | | | **Main Muscle** | **Auxiliary Muscle** |
| Leg extension | Knee extension | quadriceps | | Rectus femoris |
| | | | Vastus medialis | |
| | | | Vastus intermedius | |
| Seated leg curl | Knee flexion | Hamstring | Biceps femoris | Gastrocnemius |
| | | | Semitendinosus | |
| | | | Membranosus | |
| Leg press | Knee extension | Quadriceps | Rectus femoris | Adductor magnus |
| | Hip extension | Gluteus medius | Vastus lateralis | Hamstring |
| | | | Vastus medialis | Soleus |
| | | | Vastus intermedius | Gastrocnemius |
| Inner thigh | Hip adductor | Adductor longus | Adductor longus | |
| | | Adductor brevis | Adductor brevis | |
| | | Adductor magnus | Adductor magnus | |
| | | Gracilis | Gracilis | |
| | | Pectineus | Pectineus | |
| Outer thigh | Hip abductor | Gluteus maximus | Gluteus maximus | Gluteus medius |

As a preferred embodiment of the present disclosure, when grouping the exercise equipment, the grouping unit 410 may select a plurality of grouping criteria by using at least one of the body part classification unit 430, the joint classification unit 440, and the muscle classification unit 450 and may set an order among the plurality of grouping criteria. For example, when the number of body parts and joints is selected based on the grouping criteria, the order may be set to select the body part through the body part classification unit 430 and then group multi-joint exercise equipment for the selected body part through the joint classification unit 440. As another example, after selecting the number of joints based on the grouping criteria and selecting the multi-joint exercise equipment, exercise equipment that corresponds to the body part may be selected from among the selected multi-joint exercise equipment. The body part classification unit 430 includes a super-classification unit and a sub-classification unit. The super-classification unit classifies the body into an upper body, a lower body, and a torso, and groups exercise equipment corresponding to each body part. The sub-classification unit classifies the upper body into sub-groups: back, back of shoulder, chest, front of shoulder, biceps brachii, and triceps brachii. The lower body is classified into hips, front of the thigh, back of the thigh, inner thigh, outer thigh, calf, sole, shin, and top of the foot. Then, the torso is classified into front of the neck, back of the neck, abdomen, and waist. The sub-classification unit groups exercise equipment corresponding to the body parts classified into the sub-groups.

The joint classification unit 440 groups exercise equipment using joint information associated with each exercise equipment in the smart gym. The joint information includes joints, joint movement, number of joints, and the like used during exercise.

As a preferred embodiment of the present disclosure, the joint classification unit 440 may classify exercise equipment in the smart gym according to the number of joints used when using the exercise equipment. For example, assume that the smart gym includes seated leg press, long pull, chest press, shoulder press, seated row, and leg extension. The joint classification unit 440 classifies the seated leg press and long pull as three-joint exercise equipment. The joint classification unit 440 classifies the chest press and the shoulder press as two-joint exercise equipment. The joint classification unit 440 groups the seated row and the leg extension as single-joint exercise equipment.

As a preferred embodiment of the present disclosure, the joint classification unit 440 may also classify exercise equipment based on joint movements used when using the exercise equipment. For example, the leg extension and the leg press are grouped as exercise equipment related to knee extension.

The muscle classification unit 450 groups exercise equipment using main muscle and auxiliary muscle information associated with each exercise equipment in the smart gym. The muscle classification unit 450 groups the exercise equipment in the smart gym based on the exercise target muscle (main muscle), as shown in an example in Table 4. For example, when the exercise target muscle is vastus medialis, the leg extension and the leg press are grouped.

The exercise sequence generating unit 420 selects at least one piece of exercise equipment belonging to each of the plurality of exercise groups grouped by the grouping unit 410 and generates an exercise sequence including a use order of the at least one piece of exercise equipment and an exercise program for each of the at least one piece of exercise equipment. The exercise sequence generating unit 420 selects at least one piece of exercise equipment based on the user's physical condition and generates an exercise program for each of the at least one piece of the exercise equipment. The exercise program may include information, such as a target weight, reps, and an exercise volume for each exercise equipment.

The user's physical condition may be analyzed based on at least one of PMW, maximum oxygen intake (VO₂Max), the user's physical development status, exercise rest period, the user's subjective exercise fatigue, the user's exercise record, and the user's cumulative exercise record over a certain period.

An example of analyzing the user's physical condition using a PMW value is as follows. The exercise sequence generating unit 420 may determine the physical development status of muscles used when using the exercise equipment by using PMWₑₛₜᵢₘₐₜₑ and PMW_{individual}, each identified for each exercise equipment. For example, based on PMW_{individual} and PMWₑₛₜᵢₘₐₜₑ for leg extension, it is determined whether the quadriceps, which is the exercise target muscle of leg extension, is developed. When the PMW_{individual} is higher than the PMWₑₛₜᵢₘₐₜₑ, it is determined as developed, and when the PMW_{individual} is less than the PMWₑₛₜᵢₘₐₜₑ, it is determined as underdeveloped.

As a preferred embodiment of the present disclosure, the exercise sequence generating unit 420 may generate an exercise sequence to balance the user's physical development status by increasing the frequency of selecting exercise equipment, related to muscles for which PMW_{individual} is less than the PMWₑₛₜᵢₘₐₜₑ, from each of the plurality of exercise groups for the exercise target muscle.

In addition, the exercise sequence generating unit 420 may determine that the exercise target muscle is overdeveloped when the PMW_{individual} is higher than the preset PMWₑₛₜᵢₘₐₜₑ in each of the plurality of exercise groups for the exercise target muscle, and may generate an exercise program by selecting the intensity of exercise related to the corresponding exercise target muscle as low intensity.

When analyzing the user's physical condition using the VO₂Max, the machine learning processing unit 384 generates a percentile value for the maximum oxygen intake of the population based on user data, such as the user's gender, age, height, and weight.

In addition, based on a new user's user data, a percentile value to which the user belongs is estimated, and the corresponding maximum oxygen intake is predicted based on the data obtained from the population. When the maximum oxygen intake data obtained in a preset time unit in the new user's exercise record is greater than the predicted maximum oxygen intake, the intensity of the exercise program added based on the predicted maximum oxygen intake for the user is increased. For example, the predicted maximum oxygen intake may be increased in 5% increments. Conversely, when the maximum oxygen intake data obtained in a preset time unit in the new user's exercise record is less than the predicted maximum oxygen intake, the intensity of the exercise program added based on the predicted maximum oxygen intake for the user is decreased.

As a preferred embodiment of the present disclosure, the exercise sequence generating unit 420 provides the order of use of the exercise equipment belonging to the exercise program. The exercise sequence generating unit 420 may select exercise equipment by referring to the exercise program, user data, and exercise record of the user previously stored in the smart gym server 380. The exercise sequence generating unit 420 uses information about the main muscles and auxiliary muscles activated when the user uses the exercise equipment to generate at least one exercise sequence in a preset time unit so that the user's entire body muscles are developed evenly.

The preset exercise time unit may be 1 day, 1 week, 1 month, etc. The exercise sequence generating unit 420 may generate different exercise sequences depending on the exercise time unit.

When a user uses exercise equipment according to an exercise sequence, the smart gym server 380 collects data about exercise equipment, joints, muscles, exercise load, and movement trace when using the exercise equipment, related to the exercise sequence and stores the data as an exercise record for each user. Additionally, even when the user does not perform the entire exercise sequence but only performs part thereof, the exercise record is stored.

For example, when the grouping unit 410 groups exercise equipment into three exercise groups, namely an upper body group, a lower body group, and a torso group, for each body part, the exercise sequence generating unit 420 generates an exercise program by selecting at least one piece of the exercise equipment for each of the three exercise groups based on PMW and VO₂Max. In this case, the exercise sequence generating unit 420 provides the user with an exercise sequence including information on an order of use of the selected exercise equipment.

For example, the exercise sequence generating unit 420 may provide the user with an exercise sequence including an order of use of exercise equipment by selecting five pieces of exercise equipment related to PMW, performing an exercise program for each exercise equipment, and then using a piece of exercise equipment related to VO₂Max. In addition, among the five pieces of exercise equipment related to PMW, the order of use may be set, such as using multi-joint exercise equipment first and then using single-joint exercise equipment, depending on the user's exercise purpose and physical condition, such as injuries. Alternatively, the order of use may be set reversely.

In another preferred embodiment of the present disclosure, the exercise sequence generating unit 420 generates an exercise sequence in consideration of the user's exercise fatigue and exercise rest period. For example, when the exercise fatigue is equal to or less than a preset level, multi-joint exercise equipment may be selected from the exercise group. The exercise fatigue may be converted into a number based on the user's exercise volume accumulated over a preset period and the user's rest period. The exercise volume is calculated by multiplying exercise load by reps.

The exercise sequence generating unit 420 may set a next exercise sequence based on the user's exercise sequence performance status. For example, when the exercise time unit is one week from November 1st to 7th, four exercise sequences and a three-day rest period may be provided. When the user does not perform all four exercise sequences but only performs some thereof, or the exercise load of the exercise equipment used for each exercise sequence does not match the provided exercise sequence, the exercise sequence after January 8 may be generated by reflecting the exercise sequence performance rate performed by the user. For example, the exercise sequence generating unit 420 may select the exercise equipment to increase the frequency of use of the exercise equipment related to muscles lacking exercise by reflecting the exercise record and exercise sequence performance status from November 1 to 7.

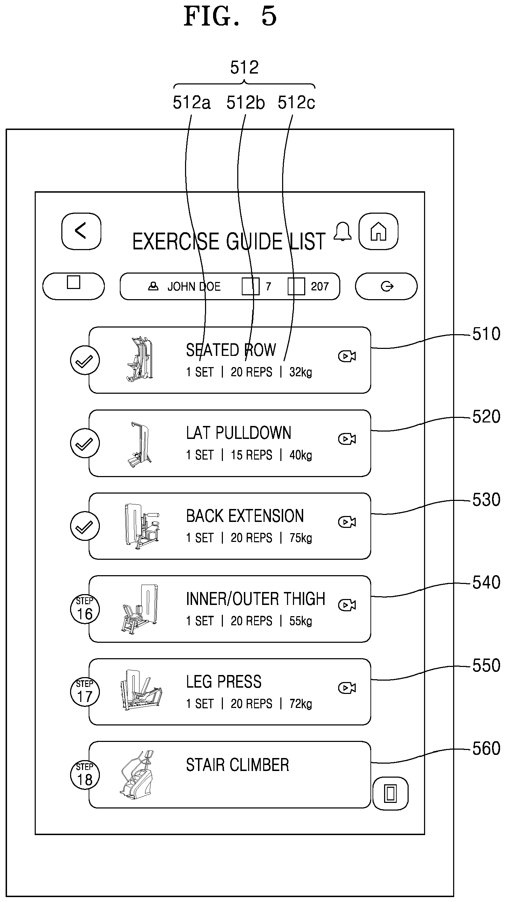

FIG. 5 is a diagram illustrating an example of providing an exercise sequence, as a preferred embodiment of the present disclosure. FIG. 5 shows part of selected 18 pieces of exercise equipment and an exercise program 512 of each exercise equipment, as a preferred embodiment of the present disclosure.

As an example of FIG. 5, an apparatus for providing an exercise sequence may provide an exercise sequence to a user terminal or a smart mirror provided in the smart gym. By tagging the user terminal to the smart mirror using NFC at the smart gym, a user may check an exercise sequence to be performed after verifying the user's membership.

FIG. 5 is an example of selecting exercise equipment for each of a plurality of exercise groups grouped by body parts by the grouping unit, displaying the order of use of selected exercise equipment (510 to 560), and displaying the exercise program 512 of each selected exercise equipment. The exercise program 512 includes the number of sets 512a, reps 512b, and a target weight 512c. The target weight 512 is initially set based on the PMWₑₛₜᵢₘₐₜₑ value, but after the preset exercise time unit has elapsed, the target weight 512 is reset based on the PMW_{individual} value reflecting the user's objectification index.

The user terminal may check in real time exercise programs that the user performed during the exercise sequence, and may also check the remaining exercise equipment and exercise programs in real time. Referring to FIG. 5, it is confirmed that the user has not used the exercise equipment (540 to 560) in steps 16 to 18 of the exercise sequence, and has performed all exercise programs of the exercise equipment (510 to 530) in the previous steps.

Methods according to embodiments of the present disclosure may be implemented in the form of program instructions that can be executed through various computer means and recorded on a computer-readable medium. The computer-readable medium may include program instructions, data files, data structures, etc., solely or in combination. The program instructions recorded on the computer-readable medium may be those specifically designed and configured for the present disclosure, or may be known and usable by those skilled in the art of computer software.

As described above, although the present disclosure has been described with reference to limited embodiments and drawings, the present disclosure is not limited to the above embodiments, and various modifications and variations can be made from these descriptions by those skilled in the art.

| |
|---|
| Industrial Applicability |
| Sequence List Text |
| PRIOR ART DOCUMENTS |
| PATENT DOCUMENTS |
| NON-PATENT DOCUMENTS |
| EXPLANATION OF REFERENCE NUMERALS DESIGNATING THE MAJOR |
| ELEMENTS OF THE DRAWINGS |

## Claims

1. An apparatus for providing an exercise sequence, the apparatus comprising:
a grouping unit for grouping exercise equipment in a smart gym into a plurality of exercise groups; and
an exercise sequence generating unit for selecting at least one piece of exercise equipment belonging to each of the plurality of exercise groups, and generating an exercise sequence including a use order of the at least one piece of exercise equipment and an exercise program for each of the at least one piece of exercise equipment.

2. The apparatus of claim 1, wherein the grouping unit performs grouping based on exercise target muscles, and in this case, further refers to joint movement or a number of joints used during exercise for each exercise target muscle.

3. The apparatus of claim 1, wherein the grouping unit performs grouping based on body parts.

4. The apparatus of claim 1, wherein the exercise sequence generating unit selects the at least one piece of exercise equipment, based on a user's physical condition, and analyzes the user's physical condition by personal maximum strength (PMW) or maximum oxygen intake (VO₂Max).

5. The apparatus of claim 4, wherein the exercise sequence generating unit generates the exercise program by setting an intensity of exercise performed for each exercise equipment belonging to the plurality of exercise groups, based on the user's physical condition, and sets the exercise intensity, based on exercise load and number of repetitions.

6. The apparatus of claim 1, wherein the exercise sequence generating unit generates at least one exercise sequence in a preset time unit so that muscles of the user's entire body are developed evenly.

7. The apparatus of claim 6, wherein the exercise sequence generating unit generates the exercise program in consideration of the user's exercise fatigue and exercise rest period, when generating the at least one exercise sequence in the preset time unit.

8. The apparatus of claim 1, further comprising a communication unit communicating with each exercise equipment in the smart gym.

9. The apparatus of claim 1, wherein the exercise sequence generating unit generates a next exercise sequence by reflecting performance results of a user performing the exercise sequence in a preset time unit.

10. A method of providing an exercise sequence, the method comprising:
grouping, by a grouping unit, exercise equipment in a smart gym into a plurality of exercise groups; and
by an exercise sequence generating unit, selecting at least one piece of exercise equipment belonging to each of the plurality of exercise groups and generating an exercise sequence including a use order of the at least one piece of exercise equipment and an exercise program for each of the at least one piece of exercise equipment.

11. The method of claim 10, wherein the exercise sequence generating unit selects the at least one piece of exercise equipment, based on a user's physical condition, and analyzes the user's physical condition by personal maximum strength (PMW) or maximum oxygen intake (VO₂Max).

12. The method of claim 11, wherein the exercise sequence generating unit generates the exercise program by setting an intensity of exercise performed for each exercise equipment belonging to the plurality of exercise groups, based on the user's physical condition, and sets the exercise intensity, based on exercise load and number of repetitions.

13. The method of claim 10, wherein the exercise sequence generating unit generates at least one exercise sequence in a preset time unit so that muscles of the user's entire body are developed evenly.

14. The method of claim 13, wherein the exercise sequence generating unit generates the exercise program in consideration of the user's exercise fatigue and exercise rest period, when generating the at least one exercise sequence in the preset time unit.

15. A computer-readable recording medium, wherein the computer-readable recording medium stores instructions that cause a computing device to perform the following method of providing an exercise sequence, the method comprising:
grouping, by a grouping unit, exercise equipment in a smart gym into a plurality of exercise groups; and
by an exercise sequence generating unit, selecting at least one piece of exercise equipment belonging to each of the plurality of exercise groups and generating an exercise sequence including a use order of the at least one piece of exercise equipment and an exercise program for each of the at least one piece of exercise equipment.
